# EUROPEAN PATENT SPECIFICATION

(11) **EP 0 896 632 B1**
(45) Date of publication and mention of the grant of the patent: **12.12.2001**
(21) Application number: 97921439.2
(22) Date of filing: 29.04.1997
(51) Int. Cl.: C12Q 1/68

(54) **METHOD FOR DETECTION AND IDENTIFICATION OF MICROORGANISMS**
VERFAHREN ZUM NACHWEIS UND ZUR IDENTIFIZIERUNG VON MIKROORGANISMEN
PROCEDE DE DETECTION ET D'IDENTIFICATION DE MICRO-ORGANISMES

(30) Priority: 01.05.1996 US 640672; 19.07.1996 US 684498; 27.02.1997 US 807138
(43) Date of publication of application: 17.02.1999
(73) Proprietor: VISIBLE GENETICS INC., Toronto, Ontario M5G 1Z6 (CA)
(72) Inventor: LACROIX, Jean-Michel, Etobicoke, Ontario M8Z 5A3 (CA); LEUSHNER, James, North York, Ontario M5M 4M3 (CA); HUI, May, Toronto, Ontario M4Y 1G1 (CA); DUNN, James, M., Scarborough, Ontario M1K 2S8 (CA); LARSON, Marina, T., Yorktown, NY 10598 (US)
(74) Representative: Wibbelmann, Jobst, Dr., Dipl.-Chem.
(86) International application number: US9707133
(87) International publication number: WO9741257

(56) References cited:
- EP-A- 0 655 506
- WO-A-92/18650
- WO-A-93/02212
- WO-A-93/08305
- US-A- 5 283 171
- ANALYTICAL BIOCHEMISTRY, vol. 216, no. 1, 1 January 1994, pages 1-14, XP000420647 VENIGALLA B RAO: "DIRECT SEQUENCING OF POLYMERASE CHAIN REACTION-AMPLIFIED DNA"
- ANALYTICAL BIOCHEMISTRY, vol. 224, no. 1, 1 January 1995, pages 117-121, XP000486746 WIEMANN S ET AL: "SIMULTANEOUS ON-LINE DNA SEQUENCING ON BOTH STRANDS WITH TWO FLUORESCENT DYES"
- J. CLIN. MICROBIOL., vol. 30, no. 9, 1992, pages 2241-2245, XP002038923 MAHONY ET AL.: "Confirmatory polymerase chain reaction testing for chlamydia trachomatis in first-void urine from asymptotic and symptomatic men" cited in the application
- NATURE, vol. 376, 31 August 1995, page 796/797 XP000606193 REEVE M A ET AL: "A NOVEL THERMOSTABLE POLYMERASE FOR DNA SEQUENCING" cited in the application
- PCR METHODS & APPLICATIONS, vol. 3, no. 5, April 1994, pages S107-S112, XP000606764 KRETZ K ET AL: "CYCLE SEQUENCING"

## Description

### BACKGROUND OF THE INVENTION

This application relates to a method for detection and identification of microorganisms, including in particular pathogenic microorganisms, and to compositions and kits useful in practicing the method. The invention can be applied to detection of viruses, including HIV and hepatitis, bacteria, including *Chlamydia*, fungi, including *Cryptococcus neoformans* and protozoa, including *Trypanosoma cruzi.*

Detection of the presence of pathogenic microorganisms through DNA-based technology is emerging as an important tool in the diagnosis of many diseases. For example, diagnosis of *Chlamydia trachomatis* infections, the most common bacterial sexually transmitted disease in North America, is shifting from traditional methods such as culture, enzyme immunoassay (EIA) and direct fluorescent antibodies (DFA) to DNA-hybridization diagnostics. Roche Diagnostic Systems, Inc. (Nutley, NJ) manufactures Amplicor™, a test which detects *C. trachomatis* and *Neisseria gonorrohoeae* by the hybridization of a pathogen specific probe to PCR amplified products, detectable by a color change/optical density technique. Abbott Laboratories (Abbott Park, IL) makes UriProbe, also a test for C. *trachomatis* and *N. gonorrohoeae*, which relies on the ligase chain reaction (LCR). The LCR method, described in Patent Applications WO 9320227, WO 9300447, WO 9408047, WO 9403636. EP 477 972 uses thermostable ligase enzyme to ligate two DNA probes which hybridize in ligatable juxtaposition on a template DNA strand, thus generating a detectable ligated DNA fragment only if the template DNA is present. A multiplex PCR assay for C. *trachomatis* has also been described in Mahony et al., *J. Clin. Microbiol.* 33: 3049-3053 (1995).

Wiemann et al., *Anal*. *Biochem*. 224: 117-121 (1995), refers to simultaneous sequencing on both strands of dsDNA using one primer for each strand, with each primer being labelled with a different fluorescent dye.

The thermal cycle sequencing method is described in Venigalla, *Anal*. *Biochem*. 216: 1-14 (1994), and in Kretz et al., *PCR Methods and Applications* 3: S107-S112.

EP-A-0 655 506 and Reeve et al., *Nature* 376: 796-797 (1995), refer to thermostable DNA polymerases.

A wide variety of infectious pathogens that can be detected by DNA-based methods are listed in *Diagnostic Molecular Microbiology,* Persing et al., eds. American Society for Microbiology, Washington D.C. (1993). This text details diagnostic tests for bacteria, virus, fungi, and protozoa. Diagnostic tests are also proposed for identifying the presence of drug resistance genes or toxin genes.

Although these tests are generally effective for identifying an infectious disease-causing organism if present, they do not routinely provide information concerning the specific serotype, variant or form of the infecting organism. Depending on the organism in question, this information can be significant in determining the likely course of the infection, for determining the most appropriate therapeutic approach and for epidemiological purposes. Furthermore, the previously known assays involve several steps and are therefore more susceptible to systematic error than would be a test with fewer steps. Thus, there remains a need for a simple test format which is generally applicable to the detection of microorganisms, including infectious disease-causing microorganisms, and particularly for a simple test which provides an indication of the specific nature, e.g., the serotype, of the organism. It is an object of the present invention to provide such a test.

It is a further object of the present invention to provide reagent combinations useful in performing tests for infectious disease-causing microorganisms, including *Chlamydia*,human papilloma virus (HPV) and HIV.

It is still a further object of the present invention to provide kits useful in performing tests for infectious disease-causing microorganisms, including *Chlamydia*, HPV and HIV.

### SUMMMARY OF THE INVENTION

The present invention provides a method for the evaluation of a sample for the presence of a target microorganism which can be performed directly on a natural abundance DNA preparation obtained from the sample in a single reaction vessel. The method of the invention comprises the steps of:
(a) combining the natural abundance DNA preparation with first and second primers, a nucleotide triphosphate feedstock mixture, a chain-terminating nucleotide triphosphate and a thermally stable polymerase enzyme which incorporates dideoxynucleotides into an extending nucleic acid polymer at a rate which is no less than 0.4 times the rate of incorporation of deoxynucleotides to form a reaction mixture, said first and second primers binding to the sense and antisense strands of the DNA of the target microorganism, respectively, and flanking a selected region within the genome of the target microorganism;
(b) exposing the reaction mixture to a plurality of temperature cycles each of which includes at least a high temperature denaturation phase and a lower temperature extension phase, thereby producing a plurality of species of terminated fragments if DNA from the target microorganism is present in the sample, each species of terminated fragment corresponding to a different incorporation position for the chain-terminating nucleotide triphosphate in the DNA of the target microorganism; and
(c) evaluating the terminated fragments produced to determine the incorporation positions of the chain-terminating nucleotide triphosphate. Based on the incorporation positions, not only the presence but also the specific nature, e.g. the serotype, of any target microorganism present can be determined.

### DETAILED DESCRIPTION OF THE INVENTION

The present invention provides a novel approach to the evaluation of a sample for the presence of a target microorganism and for the identification of the specific nature of any organism found to be present. The target microorganism may be virus, bacteria, fungi or protozoa. Specific non-limiting examples of microorganisms to which the invention can be suitably applied include bacteria such as *Mycobacteria tuberculosis, Rickettsia rickettsii*, *Ehrlichia chaffeensis, Borrelia burgdorferi, Yersinia pestis, Treponema pallidum, Chlamydia trachomatis, Chlamydia pneumoniae, Mycoplasma pneumoniae, Mycoplasma sp., Legionella pneumophila, Legionella dumoffii, Mycoplasma fermentans, Ehrlichia sp., Haemophilus influenzae, Neisseria meningitidis, Streptococcus pneumonia, S. agalactiae,* and *Listeria monocytogenes;* viruses such as Human Immunodeficiency Virus Type 1 (HIV- 1), Human T-Cell Lymphotrophic Virus Type 1 (HTLV-1), Hepatitis B Virus (HBV), Hepatitis C Virus (HCV),Herpes Simplex, Herpesvirus 6, Herpesvirus 7, Epstein-Barr Virus, Cytomegalovirus, Varicella-Zoster Virus, JC Virus, Parvovirus B19, Influenza A, B and C, Rotavirus, Human Adenovirus, Rubella Virus, Human Enteroviruses, Genital Human Papillomavirus (HPV), and Hantavirus; fungi such as *Cryptococcus neoformans, Pneumocystis carinii, Histoplasma capsulatum, Blastomyces dermatitidis, Coccidioides immitis, and Trichophyton rubrum;* and *protozoa* such as *Trypanosoma cruzi, Leishmania sp., Plasmodium, Entamoeba histolytica, Babesia microti, Giardia lamblia, Cyclospora sp.* and *Eimeria sp.* The method of the invention may also be used for *Cryplosporidium* oocyst detection; for identification of bacterial toxin genes, such as the toxin genes from *Vibrio cholerae* 01, enterotoxigenic *Escherichia coli, Shigella sp.,* enteroinvasive *E. coli, Helicobacter pylori* (formerly *Campylobacter pylori*), toxigenic *Clostridium difficile, Staphylococcus aureus,* and *Streptococcus pyogenes* exotoxins; and for identification of anti-microbial resistance loci such as rifampin resistance mutations in *Mycobacterium tuberculosis* and *M. leprae*; HIV Drug Resistance, *erm* Erythromycin Resistance Genes, methicillin--resistance genes in *Staphylococcus,* Penicillinase-Producing genes in *Neisseria gonorrhoeae*, genes encoding aminoglycoside-modifying enzymes, genes encoding an extended spectrum of Beta-Lactamases, fluoroquinolone and isoniazid resistance mutations in *Mycobacterium tuberculosis,* and genes encoding vancomycin resistance in Enterococci.

In accordance with the method of the invention, a natural abundance DNA-containing sample suspected to contain the target microorganism is combined in a reaction mixture with (1) first and second primers that hybridize with the sense and antisense strands of the DNA of the target microorganism, respectively, and flank a selected region within the genome of the target microorganism, (2) a nucleotide triphosphate feedstock mixture, (3) at least one chain-terminating nucleotide triphosphate and (4) a polymerase enzyme which incorporates dideoxynucleotides into an extending nucleic acid polymer at a rate which is no less than 0.4 times the rate of incorporation of deoxynucleotides to form a reaction mixture. This reaction mixture is processed through a plurality of thermal cycles. Each thermal cycle includes at least an extension step which is performed at a temperature of around 68 to 75 °C and a denaturation step performed at a temperature of around 90 to 98 °C. In addition, the thermal cycles may include a separate annealing step performed at a temperature of 50 to 70 °C.

During each cycle, the primers each anneal to the respective strand of any target DNA present in the sample, and primer chain extension using the polymerase enzymes and the nucleotide triphosphate feedstocks proceeds until terminated by incorporation of a chain-terminating nucleotide triphosphate. This results in the production of sequencing fragments comparable to those generated in a conventional sequencing reaction. Analysis of these fragments provides information concerning the sequence of the selected region of the target DNA, and thus of the serotype of the target microorganism. Those extension products which are not terminated prior to reaching the region complementary to the other primer can serve as template for generation of sequencing fragments in later cycles, although this generally occurs to a very small extent.

Among the advantages of the present invention is the ability to perform an evaluation directly on a "natural abundance" DNA sample. The nature of the initial sample will depend on the nature of the target microorganism. For example, in the case of *Chlamydia*, the initial sample employed in the present invention is suitably a urine sample, genital scraping or genital swab taken from a human patient, although other samples which are suspected of containing *Chlamydia* can also be tested using the method of the invention. Similarly, to test for HIV infection, the preferred sample is a blood sample.

The initial sample is treated to make DNA in the sample accessible for hybridization with the primers in the reaction mixture, for example by lysis, centrifugation to remove cell debris, and proteolytic digestion to expose the DNA. In accordance with the invention, it is not necessary to perform any type of preferential amplification of the target DNA in the sample prior to the use of the sample in the method of the invention, and indeed to reduce the number of steps and to simplify the assay it is preferred to use sample material which has not been previously subjected to any amplification procedure. As used in the specification and claims hereof, such sample materials in which the DNA in the sample has not been subjected to a preferential amplification step to increase one portion of the DNA relative to the remainder of the DNA will be referred to as "natural abundance samples." The term "natural abundance" does not, however, require the presence of all the DNA from the original sample. Thus, a sample containing just nuclear DNA, or just mitochondrial DNA or some subfraction of nuclear or mitochondrial DNA obtained by isolation from a microbial sample but not subjected to preferential amplification would be a "natural abundance" sample within the meaning of that term in the specification and claims of this application. The term "natural abundance" would also include a DNA sample prepared by conversion, for example by reverse transcription, of a total mRNA preparation or the genome of an RNA virus to cDNA; DNA isolated from an individual bacterial colony growing on a plate or from an enriched bacterial culture; and a viral DNA preparation where substantially the entire viral genome is isolated.

Primers used in the method of the present invention can be any pair of primers which hybridize with the sense and antisense strands DNA of the target microorganism flanking a selected region of diagnostic relevance, and which do not both hybridize to neighboring locations in human DNA or other microbial DNA potentially found in the sample. As used herein, the term "flanking" will be understood to mean the positioning of primers at the 5'-ends of the selected region on each DNA strand, such that extension of the primers leads to replication of the region between the primers. The primers are preferably selected such that the primer pair flanks a region that is about 500 bp or less, although primers spanning larger regions of DNA can be utilized with adjustments to the sequencing mixture (generally an increase in the relative amount of deoxynucleotide triphosphates) to increase the amount of longer sequencing fragments produced.

Primers can be selected to hybridize with highly conserved regions which are the same in all variants of the target microorganism or can be prepared as degenerate primers to take known sequence variations at the primer site into account. Thus, the first and second primers of the invention may each be a discrete oligonucleotide species, or may be a set of oligonucleotide primers with similar but not identical sequences.

Primers can also be selected to bind to the sense and antisense strands of DNA flanking a region of the genome of the target microorganism which is constant across all known variants and forms of the microorganism, in which case the method of the invention would provide detection but not any specific qualitative characterization of the microorganisms, i.e., such primers could not provide discrimination between subspecies, serovars, strains, sub-types, biovars, variants, serotypes or between closely related species of the target microorganism. An example of such a primer pair is a primer pair that binds to the cryptic plasmid of *C. trachomatis* which is recognized as a suitably specific target sequence or detection purposes, but which is not known to vary from strain to strain. Preferably, however, the primers employed will flank a region of the target genome which is variable in sequence depending on the serotype of the organism. Thus, for *C. trachomatis*, primers which flank portions of the ompl gene are preferred. Similarly, in the case of HIV detection, primers flanking known mutation sites in the HIV protease gene or reverse transcriptase gene produce fragments which permit both detection of HIV and the identification of the HIV variant present in the sample. Primers MY09 and MY11 (See example 10) give sequence information for most relevant types of human papilloma virus (HPV) but not other viruses.

In an alternative embodiment, primer pairs are selected which, when treated under the conditions of the invention, give sequence information from a much wider variety of organisms. This is the case with eubacterial "universal" primers such as 91E and 13B listed in Appendix I which can be used to obtain sequence data from the 16S rDNA gene of many bacteria. These primers are useful for identifying which bacterium is present in a septic blood culture, or any other pure but unknown culture. Patient samples which contain a broad range of bacteria will give a complex result, consisting of many overlapping sequences when tested with these primers. The complex result may, in some cases, provide useful information about the bacteria present. However, in the normal course, it is advantageous to separate out the species, i.e. by plating them out first. In this case, individual pure colonies can be selected and identified.

In still another embodiment, the primer pairs are selected to determine whether a specific gene is present in the patient sample. The gene can be a toxin gene, a virulence gene, an anti-biotic resistance gene or a specific mutation which confers drug resistance or the like. Such a test can determine if a microorganism is present and if it carries the gene at the same time.

Primers for other microorganisms can be derived from known sequence information. Appendix I lists a collection of suitable primer pairs for various other microorganisms which are taken from Persing et al., *supra.*

One or both of the primers may be labeled with a detectable label at the 5'-end thereof, particularly a fluorescent label such as fluorescein or a cyanine dye such as Cy 5.5. If labels are used on both primers, the labels selected should be spectroscopically-distinct, i.e., they should have either a different excitation spectrum or a different emission spectrum such that one primer can be distinguished from the other. When both primers are labeled with different detectable labels, the sequence of both strands of the sample can be determined in a single reaction.

The nucleotide triphosphate feedstock mixture is a standard mixture of the four conventional bases (A, C, G and T) in a buffer suitable for template-dependent primer extension with the enzyme employed. As will be appreciated by persons skilled in the art, the specific concentrations of the nucleotide triphosphates and the nature of the buffer will vary depending on the enzyme employed. Standard buffers and reagent concentrations for various known polymerase enzymes may be employed in the invention.

The reaction mixture used in the present invention also includes at least one type of chain-terminating nucleotide triphosphate. Separate reactions for the four different types of bases may be run either concurrently or successively. Running all four bases concurrently comports with conventional sequencing practice. However, a preferred embodiment of the present invention combines the single vessel methodology of this application with "single track sequencing" which is described in commonly assigned US Patent Application No. 08/577,858 (issued as US Patent No. 5,834,189). In single track sequencing, the determination of the positions of only one (or in any event less than 4) nucleotide(s) of a target sequence is frequently sufficient to establish the presence of and determine the qualitative nature of a target microorganism by providing a finger-print or bar-code of the target sequence that may be sufficient to distinguish it from all other known varieties of the sequence. Throughput is increased by reducing the number of reactions and electrophoresis runs required to identify a sequence. By selection of the order of bases tested, and intermediate analysis, it may be unnecessary to run all four bases to determine the presence and specific qualitative nature of any target microorganism present in the sample.

The polymerase enzyme used in the invention is a thermostable polymerase enzyme which incorporates dideoxynucleotides into an extending nucleic acid polymer at a rate which is no less than 0.4 times the rate of incorporation of deoxynucleotides. ThermoSequenase™ is exemplary of such an enzyme. Reeve et al., *Nature* 376: 796-797 (1995). Tabor et al. have also described enzymes which have increased processivity and increased levels of incorporation of dideoxynucleotides (See EP-A1-0 655 506, which is incorporated herein by reference).Roche sells an enzyme under the trademark TAQ-FS which meets these criteria as well.

The absolute and relative amounts of nucleotide triphosphates and chain-terminating nucleotide triphosphates may be optimized for the particular enzyme employed. In general, however, the nucleotide triphosphates will be included at in the reaction mixture at concentrations of from 250 µM to 1.5 mM, and the chain-terminating nucleotide triphosphate will be included at a level of from 0.5 µM to 30 µM to produce compositions in which the mole ratio of the chain terminating nucleotide triphosphate to the corresponding nucleotide triphosphate is from 1:50 to 1:1000, preferably from 1:100 to 1:500. This will result in incorporation of a chain-terminating nucleotide triphosphate into from 30 to 100 percent of the extending polymer chains formed during the thermal cycling of the reaction mixture.

The basic method of the invention can also be enhanced by various modifications without departing from the scope of the present invention. For example, improvements in reproducibility and sensitivity can be obtained by using a combination of an enzyme having a high affinity for incorporation of dideoxynucleotide triphosphates into the extending polymer, e.g., Thermo Sequenase™, and one having a low affinity for incorporation of dideoxynucleotide triphosphates into the extending polymer, e.g., Taq polymerase, under conditions where both enzymes are actively catalyzing template-dependent primer extension polymerization. As noted above, the high affinity enzyme produces almost entirely termination products, with very few of the polymers actually being extended to full length. On the other hand, the low affinity enzyme produces almost exclusively full length product, with relatively few termination products. Addition of the low affinity enzyme to the reaction mixture increases the sensitivity of the method by producing more full length material to be sequenced without increasing the processing time or adding processing steps. The increase in sensitivity can be controlled by varying the ratio of high affinity to low affinity enzyme present in the mixture.

It will be noted, however, that including of low affinity enzyme to produce full length product will also result in the formation of a very intense labeled full-length product peak. This peak may make analysis of the bases near the end of the sequence difficult. To obtain the benefits of increased sensitivity while making less full length product, it may be desirable to utilize a low affinity enzyme which is more thermolabile than Taq polymerase, such that the low affinity enzyme is essentially inactivated by the end of the first 15 to 25 cycles. This would allow the production of longer fragments early in the assay and the generation of more terminated fragments late in the assay.

The reaction mixture of the invention may also incorporate other additives which enhance the formation of sequencing fragments. For example, a product called TaqStart™ Antibody is a monoclonal antibody which binds to and blocks the activities of Taq polymerase. This antibody is added to PCR reactions using Taq polymerase to block enzyme activity during set-up at ambient temperature to prevent or reduce the formation of non-specific amplification products. TaqStart™ Antibody can be used in the present invention with Thermo Sequenase™ to reduce nonspecific primer extension reactions.

Other materials which can be used in the reaction mixture of the invention are uracil-DNA glycosylases and corresponding unconventional nucleotides as described in US Patent No. 5,418,149, incorporated herein by reference, to reduce non-specific product formation. Roche sells a product under the trademark AMPERASE™ which can be used conveniently for this purpose.

The method of the invention is suitably practiced using a kit which provides the appropriate reagents in conveniently packaged form. To reduce the number of sample preparation steps, and thus to reduce the risk of erroneous results, such a kit will suitably include at least one preprepared mixture comprising all four nucleotide triphosphates and at least one chain terminating nucleotide triphosphate, where the mole ratio of chain terminating nucleotide to the corresponding deoxynucleotide triphosphate is from 1:50 to 1:1000, preferably 1:100 to 1:500.

The invention will now be further described by way of the following non-limiting examples.

### EXAMPLE 1

The presence of the sexually transmitted disease pathogen *Chlamydia trachomatis* in a patient sample is detected according to the method of the invention as follows.

Urine samples from patients suspected of carrying a sexually transmitted disease pathogen are prepared for sequence- based diagnosis as follows. 100 ul of first void urine are deposited in a sterile microcentrifuge tube. The tube is centrifuged at 12,000 x g for 20 min; the supernatant is removed. 100 ul of Lysis Solution (Proteinase K @ 100 g/ml; 1% Tween 20) is added to the bacterial pellet and incubated 1 h at 55°C, or 18 h at room temperature. After a final incubation at 95°C for 10 minutes, 200 ul of Geneclean II glass milk is added, according to the manufacturer's instructions. (Bio 101, Inc) DNA is eluted in 10 ul of double distilled H₂O. (A lysis solution control may be prepared if desired, by adding the lysis solution to a sterile tube (a tube without any urine pellet), and treating this tube like the others.)

The sample natural abundance DNA is then treated according to the method of the invention with a pair of primers and reagents to identify the sequence of a *C. trachomatis* gene present in the sample, if any. A suitable *C. trachomatis* specific target for sequencing is the cryptic plasmid. Primers that may be used are

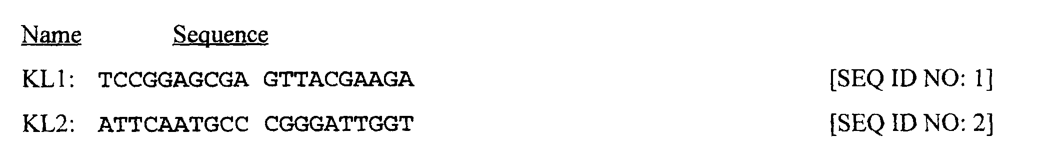

These sequencing primers were employed previously for PCR amplification reactions, but not sequencing (Mahony et al., "Confirmatory polymerase chain reaction testing for Chlamydia trachomatis in first void urine from asymptomatic and symptomatic men" *J. Clin Microbiol*. 30:2241-2245 (1992)).

Either primer may be labeled at the 5'-end with a detectable label such as a Cy5.5 fluorophore. If both primers are labeled, they should be distinguishable. Labels are selected on the basis of the instrument employed for detection. Labeling reactions are performed according to methods well known in the art, such as amidite labeling or dye-ester condensation.

The sequencing reaction mixture is prepared by combining 2.5 ul of the prepared DNA sample, 0.67 ul of 10 uM primer KL1 (labeled with Cy5.5), 0.45 ul of KL2 primer at 10 uM, 2 ul of THERMOSEQUENASE reaction buffer (250 mM Tris-HCl pH 9.0 @ 25 °C, 39 mM MgCl₂), 2 ul of THERMOSEQUENASE enzyme (Amersham Life Sciences) diluted 1/10 in the dilution buffer provided with the enzyme and 5.38 ul of double distilled H₂O. The final volume is 13 ul.

3 ul of the sequencing reaction mixture is placed in each of 4 clean tubes and covered with one drop of mineral oil (Sigma Chemical Co., Cat # M-5904). The tube is placed in a PTC-100 thermal cycler (M.J. Research, Maine) and heated for 3 min at 94°C, then cooled to 85 °C. One of the following termination mixtures are then added to each of the 4 tubes:
- 3 ul of dNTP:ddATP (1 mM each dNTP, 3.3 uM ddATP) in tube A.
- 3 ul of dNTP:ddCTP (1 mM each dNTP, 3.3 uM ddCTP) in tube C.
- 3 ul of dNTP:ddGTP (1 mM each dNTP, 3.3 uM ddGTP) in tube G.
- 3 ul of dNTP:ddTTP (1 mM each dNTP, 3.3 uM ddTTP) in tube T.

The dNTP:ddNTP mixes are preferably heated to 85 °C when added to the tube. The reaction mixture is mixed well and it is subjected to the following thermal cycling regime for 55 cycles:
94°C/30 sec.
60°C/30 sec.
70°C/1 min

After the last cycle, the tubes are kept at 70°C for 2 min, then cooled to 4°C until ready for loading. To view the reaction products, 6 ul of loading buffer (dye/stop solution) is added to each tube. The aqueous phase (the bottom phase disposed under the oil layer) is removed and put it in another tube. The sample is heated to 75 °C for 3 min, and put on ice. 2 ul of each sample is loaded in each well of a MicroGene Blaster automated DNA sequencer (Visible Genetics Inc., Toronto, ON). The reaction products are electrophoretically separated and detected. The data is analyzed using GeneObjects software (Visible Genetics Inc., Toronto, ON) to base-call (i.e. determine the DNA sequence) of the samples. The base-called sequence is compared to the known C. *trachomatis* sequence to confirm diagnosis. Results are reported to the patient file.

### EXAMPLE 2

The method of the invention may be employed to identify not only the presence of *C. trachomatis* in a patient sample but also the strain identity. Health care workers currently seek to distinguish among *Chlamydia trachomatis* strains to determine the molecular epidemiologic association of a range of diseases with infecting genotype (See Dean, D. et al "Major Outer Membrane Protein Variants of *Chlamydia trachomatis* Are Associated with Severe Upper Genital Tract Infections and Histopathology in San Francisco." J. Infect. Dis. 172:1013-22 (1995)).

A suitable strain specific target for C. trachomatis is the omp1 (outer membrane protein) gene which has at least 4 variable sequence ("VS") domains that may be used to distinguish among the 15 known genotypes of C. trachomatis (Yuan, Y et al. "Nucleotide and Deduced Amino Acid Sequences for the Four Variable Domains of the Major Outer Membrane Proteins of the 15 *Chlamydia trachomatis* Serovars" Infect. Immun. 57 1040-1049 (1989)).

Strain identification is achieved using the method of Example I with the following modifications. First of all, because of the length of the VS domains, separate reactions are performed to obtain sequence from VS1/VS2 and VS3/VS4. The following oligonucleotide primers may be employed:

For VS1/VS2:

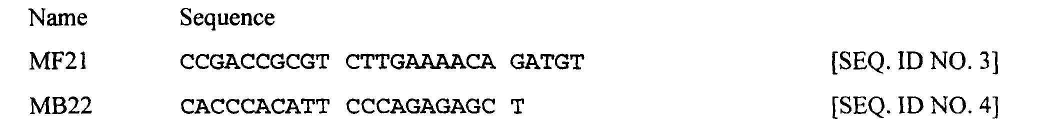

For VS3/VS4

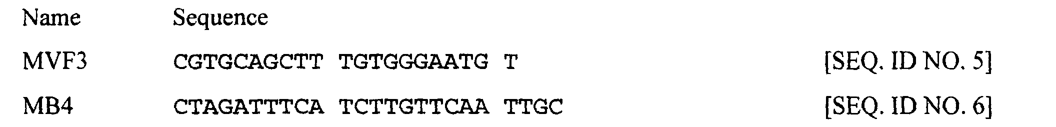

These sequencing primers were employed previously for PCR amplification reactions, but not sequencing. Mahoney et al., *supra*.

These oligonucleotide primers are used in separate reactions in place of KL 1 and KL2 in Example 1. The sample preparation and sequencing reactions are performed as in Example 1. The reaction products are electrophoretically separated and detected on a MicroGene Blaster automated DNA sequencing apparatus (Visible Genetics Inc., Toronto, ON). The data is analyzed using GeneObjects software to base-call the samples and to compare the data to the known varieties of *C. trachomatis.* Pure populations generally give unambiguous sequence data. Where heterozygous mixed populations are detected, a circumstance thought to occur in 1-3% of clinical *C. trachomatis* samples, the software identifies the strains which could be combined to result in the particular heterozygote sample detected.

### EXAMPLE 3

Strain-specific *C. trachomatis* identification over the VS1/VS2 domain can be achieved according to the method in Example 1, by using the following degenerate primers sets:

### Forward

OMP291: AGCATGCGTR TKGGTTACTA YGG [SEQ ID NO. 7]

(labeled with Cy5.5). Base 175 to 197 of the ORF of the omp1 gene of *C. trachomatis.*

### Forward

OMP314A: TGACTTTGTT TTCGACCGYG TTTT [SEQ ID NO. 8]

(labeled with Cy5.5). Base 198 to 221 of the ORF of the omp1 gene of C. trachomatis.

### Reverse

OMP722: CTAAAGTYGC RCATCCACAT TCC [SEQ ID NO. 9]

Base 637 to 615 of the ORF of the omp1 (in serovar K) gene of C. trachomatis. The primer may not have the exact same sequence as in serovar K.

### Reverse

OMP711: CATCCACATT CCCASARAGC TGC [SEQ ID NO. 10]

Base 626 to 604 of the ORF of the omp1 (in serovar K) gene of C. trachomatis. The primer may not have the exact same sequence as in serovar K.

These primers sets are preferably used in the following combinations:
(1) OMP291-OMP722, sequencing a 455 to 463-bp (depending on the serotype) fragment of the omp1 gene of C. *trachomatis*; or
(2) OMP314A-OMP711, sequencing a 421 to 430-bp (depending on the serotype) fragment of the omp1 gene of *C. trachomatis.*

### EXAMPLE 4

The method as exemplified in Examples 1, 2 and 3 may be further improved by employing different labels, preferably fluorescent labels, on the different primers for use in a multi-dye sequencer. This method takes advantage of the fact that a given termination mixture containing, for example, ddATP will give chain termination products for the A nucleotide in both directions. The different primer labels means that one reaction mixture loaded in a single lane of an automated DNA sequencing apparatus designed to detect the two labels (a "multi-dye sequencer") will identify the A nucleotide of both sense and antisense strands. Separate reactions are performed for the other 3 nucleotides. Using only 4 lanes of an electrophoresis gel, and 4 reaction mixtures, the DNA sequences of both the sense and anti-sense strands can be obtained. This information allows the operator to resolve any ambiguities that may be present.

Use of two different labels lends itself to a further improvement. As noted above, in a reaction according to the invention, the results of the ddATP reaction will give chain termination products for the A nucleotide in both directions. Since the A nucleotide in one direction corresponds to the T nucleotide in the other, a single reaction can provide the location of two bases. A second termination reaction with. for example, ddCTP will then obtain the positions of the other two nucleotides, C and G. Thus only two lanes of an electrophoresis gel and 2 reaction mixtures are required to identify the location of all 4 bases of the sequence.

A suitable multi-dye sequencer for use with this aspect of the invention, is the Applied Biosystems 377 Prism automated DNA sequencer (Applied Biosystems Inc., Foster City, CA). The fluorescent labels are selected to be detectable on the 377 instrument. Instead of the dyeterminator chemistry suggested in the Applied Biosystems product literature, however, the fluorescent labels must be conjugated to the 5' end of the primer molecules. The samples are electrophoresed, detected and the detected data is recorded.

Sophisticated software such as GeneObjects software (Visible Genetics Inc, Toronto, CA) may be used to assist in evaluation of the results. This software may employ the methods of commonly assigned US Patent Applications Nos. 08/497,202 (issued as US Patent No. 5,853,979) and 08/670,534 (issued as US Patent No. 5,916,747) and International Patent Application No. PCT/US96/11130, all of which are incorporated herein by reference. In one of the methods; the single nucleotide data tracks are evaluated and nucleotides are positioned relative to the known (or standard) DNA sequence expected from the sample. When data tracks are generated for each of the four nucleotides, the full DNA sequence of the sample may be base-called. The base-called sequence is then compared to the library of known sequences to determine which *C. trachomatis* strain or strains are present in the sample.

### EXAMPLE 5

The sequence of both the sense strand and antisense strand of a *C. trachomatis* cryptic plasmid gene may be obtained in a one step reaction using the primers:

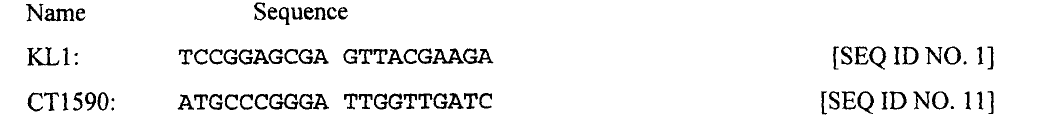

Combine the following materials and mix well:

| | Concentration | Amount |
|---|---|---|
| Patient Sample DNA | | 11.25 ul |
| KL1*Cy5.5 Primer | 10 uM | 3 ul |
| CT1590*Fluoresceine Primer | 10 uM | 2 ul |
| Enzyme Diluent (Amersham plc) | | 8 ul |
| ThermoSequenase Enzyme | 32 U/ul | 0.9 ul |
| double distilled H₂O | | 24.2 ul |

Take 11 ul of the mixture and add 2 ul of 13X buffer [Tris-HCl 260 mM pH 8.3, MgCl₂ 39 mM] (final concentration 20 mM Tris-HCl pH 8.3, 3 mM MgCl₂). Mix well and place 3 ul into each of 4 tubes. Heat tube to 94°C for 5 mins then reduce temperature to 85°C. Add and mix 3 ul of an 85 C dNTP/ddNTP solution consisting of 0.75 mM each dNTP and 2.5 uM of a chain terminating nucleotide triphosphate (ddNTP) (use a different ddNTP in each of the 4 tubes).

Treat the mixture to 60 cycles of the following thermal cycling reactions: 94°C for 10 sec, 62°C for 15 sec, 70°C for 1 min. Upon completion, treat the mixture for a final 5 min at 70 C and then store at 4°C until ready for loading. For viewing the reaction products, add an equal volume of stop/loading solution (95% formamide plus a colored dye). Take 1.5 ul and load in a single lane of a MicroGene Blaster automated DNA sequencer (Visible Genetics Inc., Toronto). Load the remaining mixture (@ 10.5 ul) in a single lane of an ALF Automated Sequencer (Pharmacia LKB, Uppsala, Sweden). The reaction products from the Cy5.5 labeled primer are detected on the MicroGene Blaster using GeneObjects Software. The reaction products from the fluorescein labeled primer are detected on the ALF Automated Sequencer using GeneObjects Software. The base-calling results of the Cy5.5 labeled primer were compared to the known sequence of the gene by the GeneLibrarian component of GeneObjects.

### EXAMPLE 6

As described in U.S. Patent Application Serial No. 08/577,858 (issued as US Patent No. 5,834,189), not all 4 nucleotides of
*C. trachomatis*, or any polymorphic or multiple allelic locus of any gene or organism necessarily need to be determined in order to ascertain which allele or variant is present. In many cases, positioning less than four nucleotides may be sufficient to determine with certainty which allele is present. The method of Examples 1 - 4 may be modified to obtain single nucleotide data tracks (or fragment patterns) by performing only one of the termination reactions at a time.

In the case of detection and serotyping of *C. trachomatis*, the evaluation of the A track alone over the first 100 nucleotides of the *ompI* gene, aligning to nucleotides 249-349 of the serovars C and K, can distinguish the serovars. Appendix II is a text file representation of the *ompI* gene in each of the serovars. The sequences are all aligned to the last (3') nucleotide of the detectably labeled primer omp314A. (Appendix II shows sequences starting 29 bp downstream of the 3'-nucleotide.) This illustration differs from a traditional "consensus" sequence illustrations in that all missing bases (usually represented by N's or raised dashes) are deleted. The A's are illustrated in the order and positions in which they would be expected to appear after a sequencing reaction and upon detection by an automated DNA electrophoresis apparatus.

If, in another microorganism, the A lane (or other preferred first lane) were not sufficient to distinguish all types, a second reaction for the C, G or T nucleotide could be performed to further define the qualitative nature of any target microorganism present in the sample. Because the sequences of the types are previously known, the operator can determine which of the nucleotides provide the greatest information and will analyze those nucleotides first.

### EXAMPLE 7

The presence of and strain identity of *C. trachomatis* in a patient sample may be determined according to the methods of the previous examples by substituting the following primer pairs. These primers are used to determine the sequence of the omp1 gene (publicly available at DNASIS Accession No. X62921).
Forward Primer (5' Primer) labeled with a detectable label such as Cy5.5: Primer OMP312: GGAGACTTTG TTTTCGACCG [SEQ ID NO 12] Position 312-331 of X62921
and one of the following Reverse Primers (3' Primer) (optionally labeled with a detectable label different from the 5' primer): Primer OMP708:    CATTCCCACA  AAGCTGCGCG    [SEQ ID NO 13] Position 727-708 of X62921 Primer OMP706: TTCCCACAAA GCTGCGCGAG [SEQ ID NO 14] Position 725-706 of X62921 Primer OMP704:	CCCACAAAGC TGCGCGAGCG	[SEQ ID NO 15] Position 723-704 of X62921

The following combination can be used to obtain DNA sequence over the following maximum lengths:
OMP312-OMP708: 416-nt region of omp1
OMP312-OMP706: 414-nt region of omp1
OMP312-OMP704: 412-nt region of omp1

### EXAMPLE 8

The presence of and strain identity of *C. trachomatis* in a patient sample may be determined according to the method of previous examples, using *C. trachomatis* ribosomal DNA (rDNA) specific primers such as CT220 ACCTTTCGGT TGAGGGAGAG TCTA [SEQ ID NO 16] and CT447 GGACCAATTC TTATTCCCAA GCGA [SEQ ID NO 17] Haydock et al., Chap 1.10 in Persing et al., *supra*.

### EXAMPLE 9

The sequence of both the sense strand and antisense strand of the protease gene of HIV- I integrated into natural abundance DNA of lymphocytes may be obtained in a one step reaction as follows.

Natural abundance DNA is prepared from the patient blood lymphocyte sample according to a standard method such as a standard salting-out procedure (as provided by the Puregene DNA Isolation Kit, Gentra Systems, Inc., Minneapolis) or by detergent and proteinase K treatment (Current Protocols in Molecular Biology, Eds. Ausubel, F.M. et al, (John Wiley & Sons; 1995)).

Combine the following materials and mix well:

| | Concentration | Amount |
|---|---|---|
| Patient Sample DNA | | 11.25 ul |
| PR211F*Cy5.5 Primer or | 10 uM | 3 ul |
| PR281*Cy5.5 Primer | 10 uM | 3 ul |
| PR526*Fluorescein Primer | 10 uM | 2 ul |
| Enzyme Diluent (Amersham plc) | | 8 ul |
| THERMOSEQUENASE Enzyme | 32 U/ul | 0.9 ul |
| double distilled H2O | | 24.2 ul |

The primers have the following sequences:

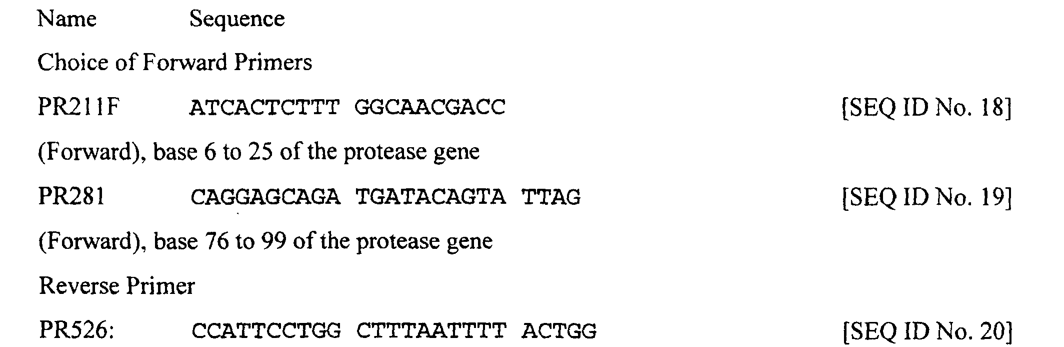

(Reverse), bases 321 to 345 of the protease gene.

PR211F-PR526 creates a sequencing fragment of maximum size 340 bp. PR281-PR526 creates a sequencing fragment of maximum size 270 bp. Both regions contain the sequence of the various codons where mutations are involved in protease inhibitor resistance (Codons 46, 48, 54, 63 82 84 and 90).

Take 11 ul of the mixture and add 2 ul of 13X buffer [Tris-HCl 260 mM pH 8.3, MgCl₂ 39 mM] (final concentration 20 mM Tris-HCl pH 8.3, 3 mM MgCl₂). Mix well and place 3 ul into each of 4 tubes. Heat tube to 94 C for 5 mins then reduce temperature to 85 C. Add and mix 3 ul of an 85 C dNTP/ddNTP solution consisting of 0.75 mM each dNTP and 2.5 uM of a chain terminating nucleotide triphosphate (ddNTP) (use a different ddNTP in each of the 4 tubes).

Treat the mixture to 60 cycles of the following thermal cycling reactions: 94 C for 10 sec, 62 C for 15 sec, 70 C for 1 min. Upon completion, treat the mixture for a final 5 min at 70 C and then store at 4 C until ready for loading. For viewing the reaction products, add an equal volume of stop/loading solution (95% formamide plus a coloured dye). Take 1.5 ul and load in a single lane of a MicroGene Blaster automated DNA sequencer (Visible Genetics Inc., Toronto). Load the remaining mixture (@ 10.5 ul) in a single lane of an ALF Automated Sequencer (Pharmacia LKB, Uppsala, Sweden). The reaction products from the Cy5.5 labeled primer are detected on the MicroGene Blaster using GeneObjects Software. The reaction products from the fluorescein labeled primer are detected on the ALF Automated Sequencer using GeneObjects Software. The base-called results from each primer were compared to the known sequences of HIV- 1 by GeneLibrarian (a component of GeneObjects (Visible Genetics Inc, Toronto).

### EXAMPLE 10

The presence and type of human papilloma virus (HPV) present in a patient sample can be determined according to the method of the invention by following the protocol in Example 1 with the following modifications.

Patient sample DNA is extracted from 250 ul urine specimens using Geneclean II (Bio 101, Inc.). The sample is then treated as described previously but employing the degenerate primer pair:

### Forward Primer: MY11

GCMCAGGGWC ATAAYAATGG [SEQ ID No. 21]

### Reverse Primer: MY09

CGTCCMAARG GAWACTGATC [SEQ ID No. 22]

The reactions are performed as before, using ThermoSequenase enzyme or the like. Reaction products are detected on an automated electrophoresis/detection device such as the MicroGene Blaster. The sequence is analyzed and compared to the known varieties of HPV to identify the type. The result is reported to the patient file.

### APPENDIX I

Suitable Sequencing Primer Pairs for Identification and Sub-Typing of Infectious Pathogens
cf. Diagnostic Molecular Microbiology (Eds. Persing et al.) (1993; American Society for Microbiology; Washington D.C.)

Bacterial Pathogens Universal (16S rDNA) Typing Primers

Pathogen Name: Universal Bacterial Identification
Gene: 16s rDNA
Forward Primer: 91E TCAAAKGAAT TGACGGGGC [SEO ID No. 23] Site of Specific Hybridization: nt 911-930
Reverse Primer:13B AGGCCCGGGA ACGTATTCAC [SEQ ID No. 24] Site of Specific Hybridization: nt 1390-1371
Maximum fragment size: 475 nt

Pathogen Name: Universal Bacterial Identification
Gene: 16s rDNA
Forward Primer: 515FPL TGCCAGCAGC CGCGGTAA [SEQ ID No. 25] Site of Specific Hybridization: nt 515-533
Reverse Primer:806R GGACTACCAG GGTATCTAAT [SEQ ID No. 26] Site of Specific Hybridization: nt 806-787
Maximum fragment size: 328 nt

Pathogen Name: Universal Bacterial Identification
Gene: 16s rDNA
Forward Primer: 11E GAGGAAGGTG GGGATGACGT [SEQ ID No. 27] Site of Specific Hybridization: nt 1175-1194
Reverse Primer: 13B AGGCCCGGGA ACGTATTCAC [SEQ ID No. 28] Site of Specific Hybridization: nt 1390-1371
Maximum fragment size: 233 nt

Pathogen Name: Eubacterial Typing (Broad range of eubacteria)
Gene: 16S rDNA
Forward Primer :285 GAGAGTTTGA TCCTGGCTCA G [SEQ ID No. 29] Site of Specific Hybridization: nt 9-30
Reverse Primer: 244 CCCACTGCTG CCTCCCGTAG [SEQ ID No. 30] Site of Specific Hybridization: nt 341-361
Maximum fragment size: 352 bp

Bacteria
Pathogen Name: Mycobacteria Typing ( M. tuberculosis complex)
Gene: 16S rDNA
Forward Primer:285 GAGAGTTTGA TCCTGGCTCA G [SEQ ID No. 31] Site of Specific Hybridization: nt 9-30
Reverse Primer:259 TTTCACGAAC AACGCGACAA [SEO ID No. 32] Site of Specific Hybridization: nt 590-609
Maximum fragment size: 600 bp

Pathogen Name: Mycobacterium tuberculosis
Gene: IS6110
Forward Primer:T5 CTCGTCCAGC GCCGCTTCGG [SEQ ID No. 33] Site of Specific Hybridization: nt 758-788
Reverse Primer:T4 CCTGCGAGCG TAGGCGTCGG [SEQ ID No. 34] Site of Specific Hybridization: nt 881-862
Maximum fragment size: 123 bp

Pathogen Name: Rickettsia rickettsii (Rocky Mountain spotted fever)
Gene: 17 K Da Ag Gene
Forward Primer:TZ15 TTCTCAATTC GGTAAGGC [SEQ ID No. 35] Site of Specific Hybridization: nt 191-209
Reverse Primer :TZ16 ATATTGACCA GTGCTATTTC [SEO ID No. 36] Site of Specific Hybridization: nt 437-419
Maximum fragment size: 247 bp

Pathogen Name: Ehrlichia chaffeensis
Gene: 16s rDNA
Forward Primer: HE1 CAATTGCTTA TAACCTTTTG GTTATAAAT [SEQ ID No. 37] Site of Specific Hybridization: nt 49-77
Reverse Primer:HE3 TATAGGTACC GTCATTATCT TCCCTAT [SEQ ID No. 38] Site of Specific Hybridization: nt 438-412
Maximum fragment size: 390 bp

Pathogen Name: Borrelia burgdorferi (Lyme disease)
Gene: Outer Surface Protein A
Forward Primer:OSPA149 TTATGAAAAA ATATTTATTG GGAAT [SEO ID No. 39] Site of Specific Hybridization: nt 1
Reverse Primer: OSPA319 CTTTAAGCTC AAGCTTGTCT ACTGT [SEQ ID No. 40] Site of Specific Hybridization: nt 193
Maximum fragment size: 193 bp

Pathogen Name: Borrelia burgdorferi (Lyme disease)
Gene: Outer Surface Protein A
Forward Primer:OSPA4 CTGCAGCTTG GAATTCAGGC ACTTC [SEQ ID No. 41] Site of Specific Hybridization: nt 638
Reverse Primer : OSPA2 GTTTTGTAAT TTCAACTGCT GACC [SEQ ID No. 42] Site of Specific Hybridization: nt 793
Maximum fragment size: 156 bp

Pathogen Name: Borrelia burgdorferi (Lyme disease)
Gene: 16s rDNA
Forward Primer:DD06 ATCTGTTACC AGCATGTAAT [SEQ ID No. 43] Site of Specific Hybridization: nt 1105
Reverse Primer:DD02 CCCTCACTAA ACATACCT [SEQ ID No. 44] Site of Specific Hybridization: nt 1472
Maximum fragment size: 368 bp

Pathogen Name: Borrelia burgdorferi (Lyme disease)
Gene: Flagellin
Forward Primer:FLA1 GATGATGCTG CTGGCATGGG AGTTTCTGG [SEQ ID No. 45] Site of Specific Hybridization: nt 121
Reverse Primer: FLA3 CTGTCTGCAT CTGAATATGT GCCGTTACCT G [SEQ ID No. 46] Site of Specific Hybridization: nt 320
Maximum fragment size: bp

Pathogen Name: Yersinia pestis (the bubonic plague)
Gene: 9.5 kb pesticin plasmid
Forward Primer: Yp1 ATCTTACTTT CCGTGAGAAG [SEQ ID No. 47] Site of Specific Hybridization: nt 971-990
Reverse Primer: Yp2 CTTGGATGTT GAGCTTCCTA [SEQ ID No. 48] Site of Specific Hybridization: nt 1450-1431
Maximum fragment size: 478 bp

Pathogen Name: Treponema pallidum (venereal syphilis)
Gene: 47-kDA gene
Forward Primer: 47-3 TTGTGGTAGA CACGGTGGGT AC [SEQ ID No. 49] Site of Specific Hybridization: nt 692 - 713
Reverse Primer: 47-4 TGATCGCTGA CAAGCTTAGG CT [SEQ ID No. 50] Site of Specific Hybridization: nt 1187-1166
Maximum fragment size: 496 bp

Pathogen Name: Treponema pallidum (venereal syphilis)
Gene: 16S rDNA
Forward Primer: Tpr3 CTCAGAGATG AGCCTGCGAC CATT [SEQ ID No. 51] Site of Specific Hybridization: nt 230
Reverse Primer: TPr4 GCATTCCCTC CCGTCCTCAT TCTTC [SEO ID No. 52] Site of Specific Hybridization: nt 480
Maximum fragment size: 251 bp

Pathogen Name: Chlamydia trachomatis (infection of mucosal surfaces)
Gene: MOMP
Forward Primer: CT.0005 GATAGCGAGC ACAAAGAGAG CTAA [SEQ ID No. 53] Site of Specific Hybridization: nt 67
Reverse Primer: CT.06 TTCACATCTG TTTGCAAAAC ACGGTCGAAA ACAAAG [SEQ ID No. 54] Site of Specific Hybridization: nt 347
Maximum fragment size: 281 bp

Pathogen Name: Chlamydia pneumoniae (respiratory disease)
Gene: 474bp PST fragment
Forward Primer: HL-1 GTTGTTCATG AAGGCCTACT [SEQ ID No. 55] Site of Specific Hybridization: nt 30-49
Reverse Primer: HR-1 TGCATAACCT ACGGTGTGTT [SEQ ID No. 56] Site of Specific Hybridization: nt 467-448
Maximum fragment size: 438 bp

Pathogen Name: Mycoplasma pneumoniae (respiratory disease)
Gene: genomic
Forward Primer: MP5-1 GAAGCTTATG GTACAGGTTG G [SEQ ID No. 57] Reverse Primer: MP5-2 ATTACCATCC TTGTTGTAAG [SEQ ID No. 58] Maximum fragment size: 144 bp

Pathogen Name: Mycoplasma speciation (Universal Primers for 8 most common Mycoplasma species)
Gene: 16S rDNA
Forward Primer: Primer A GGCGAATGGG TGAGTAACAC G [SEQ ID No. 59] Site of Specific Hybridization: nt 87
Reverse Primer: Primer B CGGATAACGC TTGCGACCTA TG [SEQ ID No. 60] Site of Specific Hybridization: nt 550
Maximum fragment size: 464 bp

Pathogen Name: Legionella pneumophila (wound infection,
respiratory disease)
Gene:
Forward Primer: LEG1 GCTATGAGGA ATCTCGCTG [SEQ ID No. 61] Reverse Primer:LEG2 CTGGCTTCTT CCAGCTTCA [SEQ ID No. 62] Maximum fragment size: 800 bp

Pathogen Name: Legionella dumoffii (wound infection, respiratory disease)
Gene:
Forward Primer: LDBKS1 ATACACGTGG TGGAGGTAC [SEQ ID No. 63] Reverse Primer: LDBKS2 GCGGGCAATA TCTTGCATC [SEQ ID No. 64] Maximum fragment size: 1000 bp

Pathogen Name: Mycoplasma fermentans
Gene: IS-like element
Forward Primer:RW005 GGTTATTCGA TTTCTAAATC GCCT [SEQ ID No. 65] Site of Specific Hybridization: nt 1116
Reverse Primer:RW004 GGACTATTGT CTAAACAATT TCCC [SEQ ID No. 66] Site of Specific Hybridization: nt 1321
Maximum fragment size: 206 nt

Pathogen Name: Ehrlichia
Gene: 16S rDNA
Forward Primer: 8F AGTTTGATCA TGGCTCAG [SEQ ID No. 67] Site of Specific Hybridization: nt 32
Reverse Primer:GA1UR GAGTTTGCCG GGACTTCTTC T [SEQ ID No. 68] Site of Specific Hybridization: nt (about 400)
Maximum fragment size: (about 400 nt)

Viruses

Human Immunodeficiency Virus Type 1 (HIV-1)
Gene: gag
Forward Primer:SK462 AGTTGGAGGA CATCAAGCAG CCATGCAAAT [SEQ ID No. 69] Site of Specific Hybridization1366-1395: nt
Reverse Primer:SK431 TGCTATGTCA GTTCCCCTTG GTTCTCT [SEQ ID No. 70] Site of Specific Hybridization: nt 1507-1481
Maximum fragment size: 142 nt

Human T-Cell Lymphotrophic Virus Type 1 (HTLV-1)
Gene: POL
Forward Primer:POL1 CCCGGGCCCC CTGACTTGTC [SEQ ID No. 71] Site of Specific Hybridization: nt 2802-2821
Reverse Primer:POL3 GCTTTCACTG TCCCACAGCAG [SEQ ID No. 72] Site of Specific Hybridization: nt 2916-2936
Maximum fragment size: 237 nt

Hepatitis B Virus (HBV)
Gene: surface antigen
Forward Primer:Primer 1 CAAGGTATGT TGCCCGTTTG [SEQ ID No. 73] Site of Specific Hybridization: nt 329-348
Reverse Primer:Primer 2 AAAGCCCTGC GAACCACTGA [SEQ ID No. 74] Site of Specific Hybridization: nt 587-568
Maximum fragment size: 259 nt

Hepatitis C Virus (HCV)
Gene: 5'UT
Forward Primer:5PUT c1-a CCCAACACTA CTCGGCTAG [SEQ ID No. 75] Site of Specific Hybridization: nt -74--92
Reverse Primer:5PUT 1-s AACTACTGTC TTCACGCAGA AAGC [SEQ ID No. 76] Site of Specific Hybridization: nt -266--289
Maximum fragment size: 216 nt

Herpes simplex virus (HSV)
Gene: DNA polymerase gene
Forward Primer:HSV-3 TACATCGGCG TCATCTGCGG GG [SEQ ID No. 77] Site of Specific Hybridization: nt 2821-2842
Reverse Primer:HSV-4 CAGTTCGGCG GTGAGGACAA AG [SEQ ID No. 78] Site of Specific Hybridization: nt 3090-3111
Maximum fragment size: 290 nt

Herpesvirus 6
Gene:
Forward Primer:H6-6 AAGCTTGCAC AATGCCAAAA AACAG [SEQ ID No. 79] Reverse Primer:H6-7 CTCGAGTATG CCGAGACCCC TAATC [SEQ ID No. 80] Maximum fragment size: 223 nt

Herpesvirus 7
Gene:
Forward Primer:HV7 TATCCCAGCT GTTTTCATAT AGTAAC [SEQ ID No. 81] Reverse Primer: HV8 GCCTTGCGGT AGCACTAGAT TTTTTG [SEQ ID No. 82] Maximum fragment size: 186 nt

Epstein-Barr Virus
Gene: EBNA2
Forward Primer:E2p1 AAGGATGCCT GGACACAAGA [SEQ ID No. 83] Site of Specific Hybridization: nt 1813-1833
Reverse Primer:E2p2 TGGTGCTGCT GGTGGTGGCA AT [SEQ ID No. 84] Site of Specific Hybridization: nt 2409-2388
Maximum fragment size: 596 nt

Cytomegalovirus (CMV) (member of Herpesviridae)
Gene: CMV IE gene
Forward Primer:CMV1 CCTAGTGTGG ATGACCTACG GGCCA [SEQ ID No. 85] Site of Specific Hybridization: nt 1234-1258
Reverse Primer:CMV2 CAGACACAGT GTCCTCCCGC TCCTC [SEQ ID No. 86] Site of Specific Hybridization: nt 1459-1483
Maximum fragment size: 249 nt

Varicella-Zoster Virus (VZV) (for Chicken Pox)
Gene: unique genomic fragment
Forward Primer:VZ7 ATGTCCGTAC AACATCAACT [SEQ ID No. 87] Site of Specific Hybridization: nt 3377-3396
Reverse Primer:VZ8 CGATTTTCCA AGAGAGACGC [SEQ ID No. 88] Site of Specific Hybridization: nt 3643-3624
Maximum fragment size: 267 nt

JC Virus (JCV) distinguishing from BK virus and simian virus 40
Gene: T antigen
Forward Primer:P5 AGTCTTTAGG GTCTTCTACC [SEQ ID No. 89] Site of Specific Hybridization: nt 4255-4274
Reverse Primer:P6 GGTGCCAACC TATGGAACAG [SEQ ID No. 90] Site of Specific Hybridization: nt 4427-4408
Maximum fragment size: 172 nt

Parvovirus B19
Gene: VP Protein
Forward Primer:Z GGAACAGACT TAGAGCTTAT TC [SEO ID No. 91] Site of Specific Hybridization: nt 2537
Reverse Primer:Y GCTTGTGTAA GTCTTCACTA G [SEQ ID No. 92] Site of Specific Hybridization: nt 2774
Maximum fragment size: 259 nt

Influenza A (Orthomyxoviridae)
Gene: hemagglutinin H2
Forward Primer:AH2B CAATAGCTGG TTTTATAGAA [SEQ ID No. 93] Site of Specific Hybridization: nt 1077
Reverse Primer:AH2CII TTATCATACA GATTCTTGAC [SEQ ID No. 94] Site of Specific Hybridization: nt 1425
Maximum fragment size: 349 nt

Influenza B (Orthomyxoviridae)
Gene: Matrix Protein
Forward Primer: BMPB GAAGGCAAAG CAGAACTAGC [SEQ ID No. 95] Site of Specific Hybridization: nt 79
Reverse Primer:BMPCII TGGCCTTCTG CTATTTCAAA [SEQ ID No. 96] Site of Specific Hybridization: nt 380
Maximum fragment size: 302 nt

Influenza C (Orthomyxoviridae)
Gene: hemagglutinin
Forward Primer:CHAB GTGCAAACTG CATCTTGTGG [SEQ ID No. 97] Site of Specific Hybridization: nt 705
Reverse Primer:CHACII CTCATTTCTT GATCTCCATG [SEQ ID No. 98] Site of Specific Hybridization: nt 1145
Maximum fragment size: 441 nt

Rotavirus
Gene: vp7
Forward Primer:A2 GGACCAAGAG AAAACGTAGC [SEQ ID No. 99] Site of Specific Hybridization: nt 805
Reverse Primer:A4 GGTCACATCA TACAATTCTA ATCTAAG [SEQ ID No. 100] Site of Specific Hybridization: nt 1062
Maximum fragment size: 257 nt

Human Adenovirus
Gene: Hexon gene
Forward Primer:A2H/pcr 4R ATGACTTTTG AGGTGGATCC CATGGA [SEQ ID No. 101] Reverse Primer:A2H/pcr 1 GCCGAGAAGG GCGTGCGCAG GTA [SEQ ID No. 102] Maximum fragment size: 134 nt

Rubella Virus
Gene: 40S ssRNA
Forward Primer:Ru2 TGCTTTGCCC CATGGGACCT CGAG [SEQ ID No. 103] Site of Specific Hybridization: nt 1990-2013
Reverse Primer:Ru3 GGCGAACACG CTCATCACGG T [SEQ ID No. 104] Site of Specific Hybridization: nt 2310-2290
Maximum fragment size: 321 nt

Human Enteroviruses
Gene: 5'NTR
Forward Primer:MD91 CCTCCGGCCC CTGAATGCGG CTAAT [SEQ ID No. 105] Site of Specific Hybridization: nt 444-468
Reverse Primer:MD90 ATTGTCACCA TAAGCAGCCA [SEQ ID No. 106] Site of Specific Hybridization: nt 577-596
Maximum fragment size: 154 nt

Genital Human Papillomavirus (HPV)
Gene: L1 gene
Forward Primer:MY11 GCMCAGGGWC ATAAYAATGG [SEQ ID No. 107] Site of Specific Hybridization: nt 6582
Reverse Primer:MY09 CGTCCMAARG GAWACTGATC [SEQ ID No. 108] Site of Specific Hybridization: nt 7033
Maximum fragment size: 450 nt

Hantavirus
Gene: M segment
Forward Primer:Har M 30+ CACTGAATAA GAGGATACAA GAATGG [SEQ ID No. 109] Site of Specific Hybridization: nt 30
Reverse Primer:Har M 403- GGAGGAATAT TACATGTGCC TTT [SEQ ID No. 110] Site of Specific Hybridization: nt 403
Maximum fragment size: 374 nt

Fungi

Cryptococcus neoformans (Universal fungal primers)
Gene:
Forward Primer: ITS1 TCCGTAGGTG AACCTGCGA [SEQ ID No. 111] Reverse Primer:ITS4 TCCTCCGCTT ATTGATATGC [SEQ ID No. 112] Maximum fragment size: 600 nt

Pneumocystis carinii
Gene: 5S rDNA
Forward Primer: 5S Sense AGTTACGGCC ATACCTCAGA [SEQ ID No. 113] Reverse Primer:5S Antisense AAAGCTACAG CACGTCGTAT [SEQ ID No. 114] Maximum fragment size: 120 nt

Fungal Pathogens (Histoplasma capsulatum, Blastomyces dermatitidis, Coccidioides immitis Trichophyton rubrum)
Gene: 18S rDNA
Forward Primer:NS3 GCAAGTCTGG TGCCAGCAGC C [SEQ ID No. 115] Site of Specific Hybridization: nt 551
Reverse Primer:RDR116 CCGTCAATTC CTTTATGTTT CAGCCTT [SEQ ID No. 116] Site of Specific Hybridization: nt 1149
Maximum fragment size: 599 nt

Protozoa

Trypanosoma cruzi
Gene: kinetoplase
Forward Primer:S35 AAATAATGKA CGGGTGAGAT GCATGA [SEQ ID No. 117] Reverse Primer:S36 GGGTTCGATT GGGGTTGGTG T [SEQ ID No. 118] Maximum fragment size: 330 nt

Leishmania species
Gene: kinetoplast
Forward Primer:13A GTGGGGGAGG GGCGTTCT [SEQ ID No. 119] Reverse Primer:13B ATTTTACACC AACCCCCAGT T [SEQ ID No. 120] Maximum fragment size: 120 nt
Plasmodium (genus specific)
Gene: Nuclear small subunit rDNA
Forward Primer: 566R GGATAACTAC GGAAAAGCTG TAGC [SEQ ID No. 121] or Forward Primer: 570R CGACTTCTCC TTCCTTTAAA AGATAGG [SEQ ID No. 122] Reverse Primer:567R GTTCAAGATT AATAATTGCA ATAATCTATC CC [SEQ ID No. 123] Maximum fragment size: about 500 nt

Entamoeba histolytica (amoebic dysentery)
Gene: SSU rDNA
Forward Primer:Psp5 GGCCAATTCA TTCAATGAAT TGAG [SEO ID No. 124] Site of Specific Hybridization: nt 200
Reverse Primer:Psp3 CTCAGATCTA GAAACAATGC TTCTC [SEQ ID No. 125] Site of Specific Hybridization: nt 1075
Maximum fragment size: 876 nt

Babesia microti
Gene: SS rDNA
Forward Primer:Bab1 CTTAGTATAA GCTTTTATAC AGC [SEQ ID No. 126] Site of Specific Hybridization: nt 38-60
Reverse Primer:Bab4 ATAGGTCAGA AACTTGAATG ATACA [SEQ ID No. 127] Site of Specific Hybridization: nt 251-275
Maximum fragment size: 238 nt

Giardia lamblia
Gene: 18S rDNA gene
Forward Primer:JW1 GCGCACCAGG AATGTCTTGT [SEQ ID No. 128] Site of Specific Hybridization: nt 1251-1270
Reverse Primer:JW2 TCACCTACGG ATACCTTGTT [SEQ ID No. 129] Site of Specific Hybridization: nt 1433-1414
Maximum fragment size: 183 nt

Pathogen Name: Cryptosporidium oocyst detection
Gene: 18S rDNA
Forward Primer: CP1 CCGAGTTTGA TCCAAAAAGT TACGAA [SEQ ID No. 130] Reverse Primer: CP2 TAGCTCCTCA TATGCCTTAT TGAGTA [SEQ ID No. 131] Maximum fragment size: 452 nt

Pathogen Name: Cyclospora and Eimeria species
Gene: 18S rDNA
Forward Primer: CYC3FE GGAATTCCTT CCGAGCTTCG CTGCGT [SEQ ID No. 132] Site of Specific Hybridization: nt 685-704
Reverse Primer: CYC4RB CGGGATCCCG TCTTCAAACC CCCTACTG [SEQ ID No. 133] Site of Specific Hybridization: nt 978-959
Maximum fragment size: 294 nt

Identification of Bacterial Toxin Genes

Pathogen Name: Vibrio cholerae 01 containing cholera toxin gene (epidemic chloera)
Gene: CTXA
Forward Primer: CTX2 CGGGCAGATT CTAGACCTCC TG [SEQ ID No. 134] Site of Specific Hybridization: nt 73-94
Reverse Primer: CTX3 CGATGATCTT GGAGCATTCC CAC [SEQ ID No. 135] Site of Specific Hybridization: nt 614-636
Maximum fragment size: 564 bp

Pathogen Name: Enterotoxigenic Escherichia coli
Gene: ST1a or ST1b
Forward Primer: ST1-1 TTAATAGCAC CCGGTACAAG CAGG [SEQ ID No. 136] Site of Specific Hybridization: nt 243-266
Reverse Primer: ST1-2 CTTGACTCTT CAAAAGAGAA AATTAC [SEQ ID No. 137] Site of Specific Hybridization: nt 127-144
Maximum fragment size: 147 bp

Pathogen Name: Enterotoxigenic Escherichia coli
Gene: LT1a or LT1b
Forward Primer: LT1a/b-1 TCTCTATRTG CAYACGGAGC [SEQ ID No. 138] Site of Specific Hybridization: nt 46-65
Reverse Primer: LT1-2 CCATACTGAT TGCCGCAAT [SEQ ID No. 139] Site of Specific Hybridization: nt 349-367
Maximum fragment size: 322 bp

Pathogen Name: Enterotoxigenic Escherichia coli
Gene: SLTII
Forward Primer:SLTII-1 CTTCGGTATC CTATTCCCGG [SEO ID No. 140] Site of Specific Hybridization: nt 288-307
Reverse Primer:SLTII-2 GGATGCATCT CTGGTCATTG [SEQ ID No. 141] Site of Specific Hybridization: nt 747-766
Maximum fragment size: 478 bp

Pathogen Name: Shigella species and enteroinvasive E. coli (diarrheal disease)
Gene: Invasion plasmid ial locus
Forward Primer: Sh-1 CTGGATGGTA TGGTGAGG [SEQ ID No. 142] Reverse Primer: Sh-2 GGAGGCCAAC AATTATTTCC [SEQ ID No. 143] Maximum fragment size: 320 bp

Pathogen Name: Helicobacter pylori (formerly Campylobacter pylori)
Gene: genomic
Forward Primer: CAM-2 TAACAAACCG ATAATGGCGC [SEQ ID No. 144] Reverse Primer: CAM-4 CATCTTGTTA GAGGGATTGG [SEQ ID No. 145] Maximum fragment size: 203 bp

Pathogen Name: Toxigenic Clostridium difficile
Gene: rDNA
Forward Primer: PG-48 CTCTTGAAAC TGGGAGACTT GA [SEO ID No. 146] Reverse Primer: B CCGTCAATTC MTTTRAGTTT [SEQ ID No. 147] Maximum fragment size: 291 bp

Pathogen Name: Toxigenic Clostridium difficile
Gene: Toxin B
Forward Primer: YT-17 GGTGGAGCTT CAATTGGAGA G [SEQ ID No. 148] Reverse Primer: YT-18 GTGTAACCTA CTTTCATAAC ACCAG [SEQ ID No. 149] Maximum fragment size: 399 bp

Pathogen Name: Staphylococcus aureus toxins and virulence factors
Gene: sea
Forward Primer:SEA1 TTGGAAACGG TTAAAACGAA [SEQ ID No. 150] Site of Specific Hybridization: nt 490-509
Reverse Primer:SEA2 GAACCTTCCC ATCAAAAACA [SEQ ID No. 151] Site of Specific Hybridization: nt 610-591
Maximum fragment size: 120 bp

Pathogen Name: Staphylococcus aureus toxins and virulence factors
Gene: seb
Forward Primer:SEB1 TCGCATCAAA CTGACAAACG [SEQ ID No. 152] Site of Specific Hybridization: nt 634-653
Reverse Primer:SEB2 GCAGGTACTC TATAAGTGCC [SEQ ID No. 153] Site of Specific Hybridization: nt 1110-1091
Maximum fragment size: 478 bp

Pathogen Name: Staphylococcus aureus toxins and virulence factors
Gene: sec
Forward Primer: SEC1 GACATAAAAG CTAGGAATTT [SEQ ID No. 154] Site of Specific Hybridization: nt 676-695
Reverse Primer:SEC2 AAATCGGATT AACATTATCC [SEQ ID No. 155] Site of Specific Hybridization: nt 932-913
Maximum fragment size: 257 bp

Pathogen Name: Staphylococcus aureus toxins and virulence factors
Gene: sed
Forward Primer:SED1 CTAGTTTGGT AATATCTCCT [SEQ ID No. 156] Site of Specific Hybridization: nt 354-373
Reverse Primer:SED2 TAATGCTATA TCTTATAGGG [SEQ ID No. 157] Site of Specific Hybridization: nt 671-652
Maximum fragment size: 317 bp

Pathogen Name: Staphylococcus aureus toxins and virulence factors
Gene: see
Forward Primer:SEE1 TAGATAAAGT TAAAACAAGC [SEQ ID No. 158] Site of Specific Hybridization: nt 491-510
Reverse Primer:SEE2 TAACTTACCG TGGACCCTTC [SEQ ID No. 159] Site of Specific Hybridization: nt 659-640
Maximum fragment size: 170 bp

Pathogen Name: Staphylococcus aureus toxins and virulence factors
Gene: tss
Forward Primer:TSST1 ATGGCAGCAT CAGCTTGATA [SEQ ID No. 160] Site of Specific Hybridization: nt 251-270
Reverse Primer:TSST2 TTTCCAATAA CCACCCGTTT [SEQ ID No. 161] Site of Specific Hybridization: nt 600-581
Maximum fragment size: 350 bp

Pathogen Name: Staphylococcus aureus toxins and virulence factors
Gene: eta
Forward Primer:ETA1 CTAGTGCATT TGTTATTCAA [SEQ ID No. 162] Site of Specific Hybridization: nt 374-393
Reverse Primer:ETA2 TGCATTGACA CCATAGTACT [SEQ ID No. 163] Site of Specific Hybridization: nt 492-473
Maximum fragment size: 119 bp

Pathogen Name: Staphylococcus aureus toxins and virulence factors
Gene: etb
Forward Primer:ETB1 ACGGCTATAT ACATTCAATT [SEQ ID No. 164] Site of Specific Hybridization: nt 51-70
Reverse Primer:ETB2 TCCATCGATA ATATACCTAA [SEQ ID No. 165] Site of Specific Hybridization: nt 250-231
Maximum fragment size: 200 bp

Pathogen Name: Bacterial meningitis (Haemophilus influenzae, Neisseria meningitidis, Streptococcus pneumonia, S. agalactiae, Listeria monocytogenes, enteric bacteria, or Mycobacterium tuberculosis)
Gene: 16s rDNA
Forward Primer:RW01 AACTGGAGGA AGGTGGGGAT [SEQ ID No. 166] Reverse Primer:DG74 AGGAGGTGAT CCAACCGCA [SEQ ID No. 167] Maximum fragment size: 370 nt

Pathogen Name: Streptococcus pyogenes exotoxins (Streptococcal Toxic Shock Syndrome)
Gene: spec
Forward Primer:F CCACCTTGAC TATTT [SEQ ID No. 168] Reverse Primer:R TTAATTAGGA GGTAA [SEQ ID No. 169] Maximum fragment size: 936 nt

Identification of Anti-Microbial Resistance Loci

Pathogen Name: Rifampin Resistance Mutations in Mycobacterium tuberculosis and M. leprae
Gene: rpoB
Forward Primer:rpoB105 CGTGGAGGCG ATCACACCGC AGACGT [SEQ ID No. 170] Reverse Primer:rpoB293 AGTGCGACGG GTGCACGTCG CGGACCT [SEQ ID No. 171] Maximum fragment size: 215 nt

Pathogen Name: Human Immunodeficiency Virus Drug Resistance
Gene: HIV Reverse Transcriptase
Forward Primer:A(35) TTGGTTGCAC TTTAAATTTT CCCATTAGTC CTATT [SEQ ID No. 172] Reverse Primer:NE-1(35) CCTACTAACT TCTGTATGTC ATTGACAGTC CAGCT [SEQ ID No. 173] Maximum fragment size: 805 nt

Pathogen Name: erm Erythormycin Resistance Genes (in S. aureus, E. coli, or Bacillus sphaericus)
Gene: erm
Forward Primer:E1 
Forward Primer:EF
GARATIGGII IIGGIAARGG ICA*                    [SEQ ID No. 174]
 Reverse Primer:E2 AAYTGRTTYT TIGTRAA* [SEQ ID No. 175] Maximum fragment size: 530 nt
*I=inosine

Pathogen Name: Methicillin-Resistant Staphylococcus
Gene: mecA
Forward Primer:RSM 2647 AAAATCGATG GTAAAGGTTG GC [SEQ ID No. 176] Site of Specific Hybridization: nt 1282-1303
Reverse Primer: RSM 2648 AGTTCTGCAG TACCGGATTT GC [SEQ ID No. 177] Site of Specific Hybridization: nt 1814-1793
Maximum fragment size: 533 nt

Pathogen Name: Penicillinase-Producing Neisseria gonorrhoeae
Gene: TEM-1
Forward Primer:PPNG-L AGTTATCTAC ACGACGG [SEO ID No. 178] Reverse Primer:PPNG-R GGCGTACTAT TCACTCT [SEQ ID No. 179] Maximum fragment size: 761 nt

Pathogen Name: Aminoglycoside-Modifying Enzymes
Gene: aacC1
Forward Primer:aacC1-1 ACCTACTCCC AACATCAGCC [SEQ ID No. 180] Reverse Primer:aacC1-2 ATATAGATCT CACTACGCGC [SEQ ID No. 181] Maximum fragment size: 169 nt

Pathogen Name: Extended Spectrum of Beta-lactamases
Gene: TEM-1
Forward Primer:Lag.Std.3 AGAGAATTAT GCAGTGC [SEQ ID No. 182] Site of Specific Hybridization: nt 560
Reverse Primer:Amp. Primer 2 GACAGTTACC AATGCTTAAT CA [SEQ ID No. 183] Site of Specific Hybridization: nt 1074
Maximum fragment size: 514 nt

Pathogen Name: Fluoroquinolone Resistance mutations in
Mycobacterium tuberculosis
Gene: gyrA
Forward Primer:GrA1 CAGCTACATC GACTATGCGA [SEQ ID No. 184] Site of Specific Hybridization: nt 78-97
Reverse Primer:GVrA2 GGGCTTCGGT GTACCTCAT [SEQ ID No. 185] Site of Specific Hybridization: nt 379-397
Maximum fragment size: 320 nt

Pathogen Name: Isoniazid Resitance of Mycobacterium tuberculosis
Gene: katG
Forward Primer:katG904 AGCTCGTATG GCACCGGAAC [SEQ ID No. 186] Site of Specific Hybridization: nt 904
Reverse Primer:katG1523 TTGACCTCCC ACCCGACTTG [SEQ ID No. 187] Site of Specific Hybridization: nt 1523
Maximum fragment size: 620 nt

Pathogen Name: Vancomycin resistant Enterococci
Gene: vanB
Forward Primer:VanB1 CATCGCCGTC CCCGAATTTC AAA [SEQ ID No. 188] Reverse Primer:VanB2 GATGCGGAAG ATACCGTGGC T [SEQ ID No. 189] Maximum fragment size: 297 nt

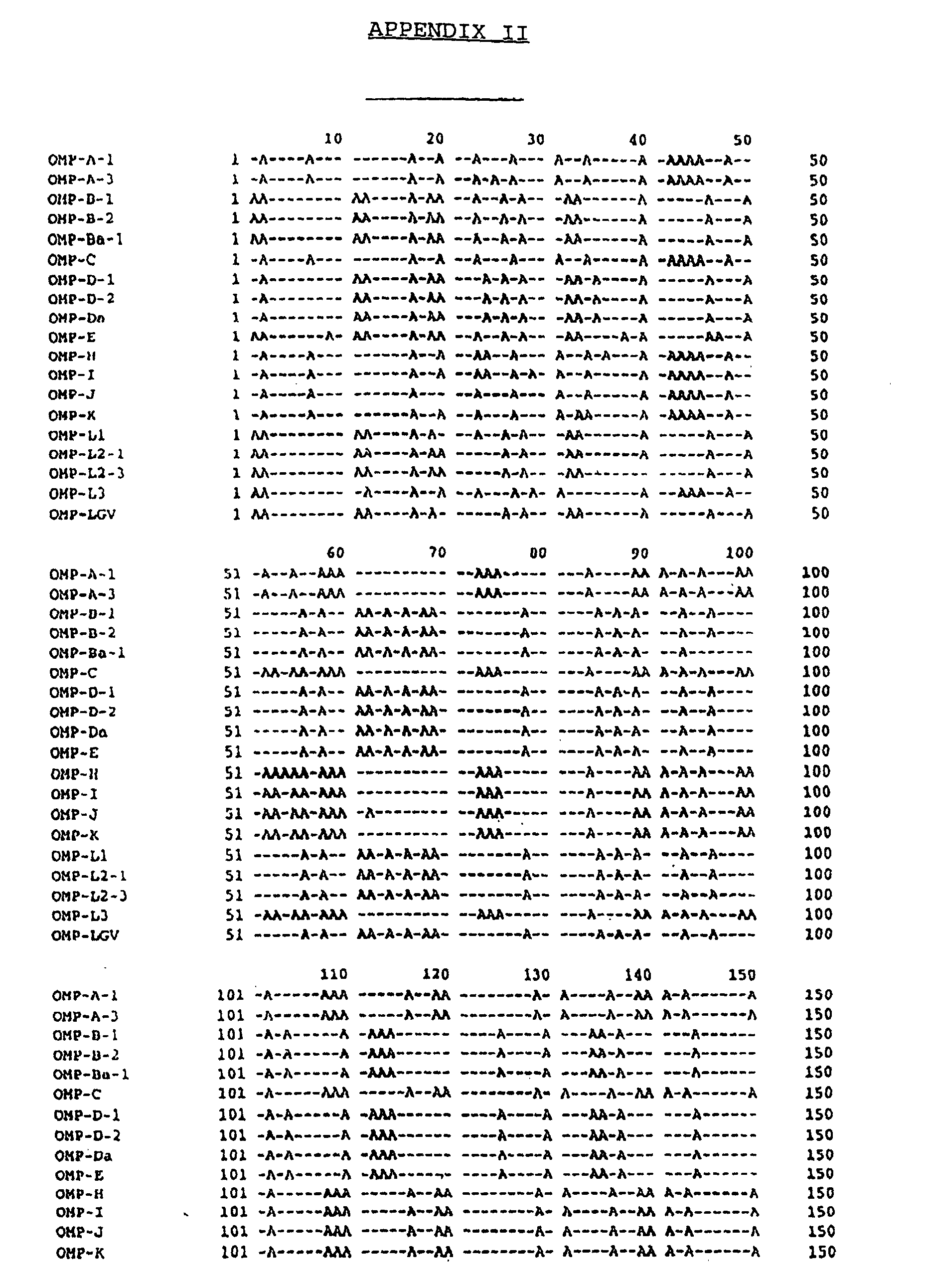

## Claims

1. A method for evaluating a natural abundance sample for the presence of DNA from a target microorganism comprising the steps of:
(a) combining the natural abundance sample with first and second primers, a nucleotide triphosphate feedstock mixture, a chain-terminating nucleotide triphosphate and a thermally stable polymerase enzyme which incorporates dideoxynucleotides into an extending nucleic acid polymer at a rate which is no less than 0.4 times the rate of incorporation of deoxynucleotides in an amplification mixture to form a reaction mixture, said first and second primers binding to the sense and antisense strands, respectively, and flanking a selected region within the target microorganism genome and at least one of said first and second primers being labeled with a fluorescent label;
(b) exposing the reaction mixture to a plurality of temperature cycles each of which includes at least a high temperature denaturation phase and a lower temperature extension phase, thereby producing a plurality of species of terminated fragments if DNA from the target microorganism is present in the natural abundance sample, each species of terminated fragment corresponding to a different incorporation position for the chain-terminating nucleotide triphosphate in the DNA of the target microorganism DNA; and
(c) evaluating the terminated fragments produced to determine the incorporation positions of the chain-terminating nucleotide triphosphate.

2. The method of claim 1, wherein the first and second primers are each labeled with a different fluorescent label.

3. The method of any of claims 1 or 2, wherein the chain terminating nucleotide triphosphate is present in a mole ratio to the corresponding nucleotide triphosphate of from 1:50 to 1:1000.

4. The method of claim 3 wherein the mole ratio of the chain terminating nucleotide triphosphate to the corresponding nucleotide triphosphate is from 1:100 to 1:500.

5. The method according to any of claims I to 4, wherein the target microorganism is *Chlamydia trachomatis*.

6. The method according to claim 5 wherein the first and second primers are selected from among the group consisting of the oligonucleotides given by Seq. ID. Nos. 1-17.

7. The method according to any of claims 1 to 4 , wherein the target microorganism is human immunodeficiency virus.

8. The method according to claim 7, wherein the first and second primers are selected from among the group consisting of the oligonucleotides given by Seq. ID. Nos. 18-20.

9. The method according to any of claims 1 to 4, wherein the target microorganism is human papilloma virus.

10. The method according to claim 9, wherein the first and second primers are selected from among the group consisting of the oligonucleotides given by Seq. ID. Nos. 21-22.

11. The method of claim 1, wherein the natural abundance sample consists of from one to three aliquots, and wherein the chain terminating nucleotide triphosphate added to each aliquot is different from that added to the other aliquots.

12. Use in a method according to any of claims 1-10 of a composition comprising a mixture of four deoxynucleotide triphosphates and at least one dideoxynucleotide triphosphate corresponding to one of the four deoxynucleotide triphosphates, wherein the dideoxynucleotide triphosphate is present in a mole ratio to the corresponding deoxynucleotide triphosphate of from 1:50 to 1:500.

13. The use according to claim 12, wherein the mole ratio is from 1:100 to 1:300.

14. The use according to claim 12 or 13, further comprising a thermally stable polymerase enzyme which incorporates dideoxynucleotides into an extending nucleic acid polymer at a rate which is no less than 0.4 times the rate of incorporation of deoxynucleotides.

15. Use in a method according to any of claims 1-10 of a kit comprising, in packaged combination,
(a) a pair of primers which bind to the sense and antisense strands, respectively, and flank a selected region within the genome target microorganism, at least one of said primers being labeled with a fluorescent label; and
(b) a mixture of four deoxynucleotide triphosphates and at least one dideoxynucleotide triphosphate corresponding to one of the four deoxynucleotide triphosphates, wherein the dideoxynucleotide triphosphate is present in a mole ratio to the corresponding deoxynucleotide triphosphate of from 1:50 to 1:1000.

16. The use according to claim 15, wherein the mole ratio is from 1:100 to 1:500.

17. The use according to claim 15 or 16, further comprising a polymerase enzyme which incorporates dideoxynucleotides into an extending nucleic acid polymer at a rate which is no less than 0.4 times the rate of incorporation of deoxynucleotides.

18. The use according to any of claim 15 to 17, wherein the primers are each labeled with a spectroscopically-distinct fluorescent label.

19. The use according to any of claims 15 to 18, wherein the target microorganism is *Chlamydia trachomatis*.

20. The use according to claim 19, wherein the first and second primers are selected from among the group consisting of the oligonucleotides given by Seq. ID. Nos. 1-17.

21. The use according to any of claims 15 to 18, wherein the target microorganism is human immunodeficiency virus.

22. The use according to claim 21, wherein the first and second primers are selected from among the group consisting of the oligonucleotides given by Seq. ID. Nos. 18-20.

23. The use according to any of claims 15 to 18, wherein the target microorganism is human papilloma virus.

24. The use according to claim 23, wherein the first and second primers are selected from among the group consisting of the oligonucleotides given by Seq. ID. Nos. 21-22.

## Patentansprüche

1. Verfahren zur Auswertung einer Probe mit natürlichem Vorkommen bezüglich der Anwesenheit von DNS aus einem Ziel-Mikroorganismus, umfassend die Schritte:
(a) Vereinigen der Probe mit natürlichem Vorkommen mit einem ersten und zweiten Primer, einer Nucleotidtriphosphat-Eduktmischung, einem Ketten-beendenden Nucleotidtriphosphat und einem thermisch stabilen Polymeraseenzym, welches Didesoxynucleotide in ein sich verlängerndes Nucleinsäurepolymer mit einer Geschwindigkeit einbaut, die nicht geringer ist als das 0,4-fache der Einbaugeschwindigkeit von Desoxynucleotiden, in einer Amplifikationsmischung, um eine Reaktionsmischung zu bilden, wobei sich der erste und der zweite Primer an den Sinn- bzw. Antisinn-Strang binden und eine ausgewählte Region innerhalb des Genoms des Ziel-Mikroorganismus flankieren und mindestens einer des ersten und des zweiten Primers mit einer fluoreszierenden Markierung markiert ist;
(b) Einwirkenlassen einer Mehrzahl von Temperaturzyklen, von denen jeder mindestens eine Hochtemperatur-Denaturierungsphase und eine Verlängerungsphase bei niedrigerer Temperatur einschließt, auf die Reaktionsmischung, wodurch eine Mehrzahl von Spezies aus abgebrochenen Fragmenten erzeugt wird, falls DNS aus dem Ziel-Mikroorganismus in der Probe mit natürlichem Vorkommen anwesend ist, wobei jede Spezies aus abgebrochenem Fragment einer unterschiedlichen Einbauposition des Ketten-abbrechenden Nucleotidtriphosphats in der DNS der Ziel-Mikroorganismus-DNS entspricht; und
(c) Auswerten der erzeugten abgebrochenen Fragmente, um die Einbaupositionen des Ketten-abbrechenden Nucleotidtriphosphats zu bestimmen.

2. Verfahren nach Anspruch 1, in dem der erste und der zweite Primer jeweils mit einer unterschiedlichen fluoreszierenden Markierung markiert sind.

3. Verfahren nach irgendeinem der Ansprüche 1 oder 2, in dem das Ketten-abbrechende Nucleotidtriphosphat in einem Molverhältnis von 1: 50 bis 1 : 1000 zu dem entsprechenden Nucleotidtriphosphat anwesend ist.

4. Verfahren nach Anspruch 3, in dem das Molverhältnis des Ketten-abbrechenden Nucleotidtriphosphats zu dem entsprechenden Nucleotidtriphosphat 1 : 100 bis 1 : 500 beträgt.

5. Verfahren nach irgendeinem der Ansprüche 1 bis 4, in dem der Ziel-Mikroorganismus *Chlamydia trachomatis* ist.

6. Verfahren nach Anspruch 5, in dem der erste und der zweite Primer aus der Gruppe ausgewählt sind, die aus den Oligonucleotiden besteht, welche durch die Seq. ID Nr. 1 - 17 gegeben sind.

7. Verfahren nach irgendeinem der Ansprüche 1 bis 4, in dem der Ziel-Mikroorganismus das Human-lmmunodeficiency-Virus ist.

8. Verfahren nach Anspruch 7, in dem der erste und der zweite Primer aus der Gruppe ausgewählt sind, die aus den Oligonucleotiden besteht, welche durch die Seq. ID. Nr. 18- 20 gegeben sind.

9. Verfahren nach irgendeinem der Ansprüche 1 bis 4, in dem der Ziel-Mikroorganismus das humane Papilloma-Virus ist.

10. Verfahren nach Anspruch 9, in dem der erste und der zweite Primer aus der Gruppe ausgewählt sind, die aus den Olignucleotiden besteht, welche durch die Seq. ID Nr. 21 - 22 gegeben sind.

11. Verfahren nach Anspruch 1, in dem die Probe mit natürlichem Vorkommen aus einer bis drei Aliquoten besteht und in dem das Ketten-abbrechende Nucleotidtriphosphat, das zu jeder Aliquote gegeben wird, von demjenigen verschieden ist, das den anderen Aliquoten zugesetzt wird.

12. Verwendung einer Zusammensetzung in einem Verfahren nach irgendeinem der Ansprüche 1 - 10, welche eine Mischung von 4 Desoxynucleotidtriphosphaten und mindestens einem Didesoxynucleotidtriphosphat umfasst, welches einem der vier Desoxynucleotidtriphosphate entspricht, wobei das Didesoxynucleotidtriphosphat in einem Molverhältnis von 1 : 50 bis 1 : 500 zu dem entsprechenden Desoxynucleotidtriphosphat vorliegt.

13. Verwendung nach Anspruch 12, in dem das Molverhältnis 1 : 100 bis 1 : 300 beträgt.

14. Verwendung nach Anspruch 12 oder 13, weiter umfassend ein thermisch stabiles Polymeraseenzym, das Didesoxynucleotide in ein sich verlängerndes Nucleinsäurepolymer mit einer Geschwindigkeit einbaut, die nicht weniger als das 0,4-fache der Einbaugeschwindigkeit von Desoxynucleotiden beträgt.

15. Verwendung eines Testsatzes in einem Verfahren nach irgendeinem der Ansprüche 1 bis 10, in abgepackter Kombination umfassend
(a) ein Paar Primer, die sich an den Sinn- bzw. Antisinn-Strang binden und eine ausgewählte Region innerhalb des Genoms des Ziel-Mikroorganismus flankieren, wobei mindestens einer der Primer mit einer fluoreszierenden Markierung markiert ist; und
(b) eine Mischung von 4 Desoxynucleotidtriphosphaten und mindestens einem Didesoxynucleotidtriphosphat, das einem der vier Desoxynucleotidtriphosphate entspricht, wobei das Didesoxynucleotidtriphosphat in einem Molverhältnis von 1 : 50 bis 1 : 1000 zu dem entsprechenden Desoxynucleotidtriphosphat vorliegt.

16. Verwendung nach Anspruch 15, bei der das Molverhältnis 1 : 100 bis 1 : 500 beträgt.

17. Verwendung nach Anspruch 15 oder 16, weiter umfassend ein Polymeraseenzym, das Didesoxynucleotide in ein sich verlängerndes Nucleinsäurepolymer mit einer Geschwindigkeit einbaut, die nicht weniger als das 0,4-fache der Geschwindigkeit des Einbaus von Desoxynucleotiden beträgt.

18. Verwendung nach irgendeinem der Ansprüche 15 bis 17, bei der die Primer jeweils mit einer spektroskopisch unterschiedlichen fluoreszierenden Markierung markiert sind.

19. Verwendung nach irgendeinem der Ansprüche 15 bis 18, bei der der Ziel-Mikroorganismus *Chlamydia trachomatis* ist.

20. Verwendung nach Anspruch 19, bei der der erste und der zweite Primer aus der Gruppe ausgewählt sind, die aus den Oligonucleotiden besteht, welche durch Seq. ID Nr. 1 - 17 gegeben sind.

21. Verwendung nach irgendeinem der Ansprüche 15 bis 18, bei der der Ziel-Mikroorganismus das Human-Immunodeficiency-Virus ist.

22. Verwendung nach Anspruch 21, bei der der erste und der zweite Primer aus der Gruppe ausgewählt sind, die aus den Oligonucleotiden besteht, welche durch Seq. ID Nr. 18- 20 gegeben sind.

23. Verwendung nach irgendeinem der Ansprüche 15 bis 18, bei der der Ziel-Mikroorganismus das humane Papilloma-Virus ist.

24. Verwendung nach Anspruch 23, in der der erste und der zweite Primer aus der Gruppe ausgewählt sind, die aus den Oligonucleotiden besteht, die durch die Seq. ID Nr. 21- 22 gegeben sind.

## Revendications

1. Procédé d'évaluation de la présence d'ADN d'un micro-organisme cible dans un échantillon d'abondance naturelle, comprenant les étapes selon lesquelles:
(a) on combine l'échantillon d'abondance naturelle avec une première et une seconde amorce, un mélange substrat de nucléotides triphosphates, un nucléotide triphosphate terminateur de chaîne et une enzyme polymérase stable à la chaleur qui incorpore des didésoxynucléotides dans un polymère acide nucléique croissant à un taux égal à au moins 0,4 fois le taux d'incorporation de désoxynucléotides dans un mélange d'amplification pour former un mélange réactionnel, lesdites première et seconde amorces se liant respectivement aux brins sens et antisens et flanquant une région choisie dans le génome du micro-organisme cible, et au moins l'une desdites première et seconde amorces étant marquée avec un marqueur fluorescent;
(b) on expose le mélange réactionnel à plusieurs cycles de température dont chacun comprend au moins une phase de dénaturation thermique et une phase d'élongation à basse température, en produisant ainsi plusieurs espèces de fragments terminés si l'ADN du micro-organisme cible est présent dans l'échantillon d'abondance naturelle, chaque espèce de fragment terminé correspondant à une position d'incorporation différente pour le nucléotide triphosphate terminateur de chaîne dans l'ADN du micro-organisme cible; et
c) on évalue les fragments terminés produits pour déterminer les positions d'incorporation du nucléotide triphosphate terminateur de chaîne.

2. Procédé selon la revendication 1, dans lequel la première et la seconde amorce sont marquées chacune avec un marqueur fluorescent différent.

3. Procédé selon l'une quelconque des revendications 1 ou 2, dans laquelle le nucléotide triphosphate terminateur de chaîne est présent en un rapport molaire au nucléotide triphosphate correspondant compris entre 1:50 et 1:1 000.

4. Procédé selon la revendication 3, dans lequel le rapport molaire du nucléotide triphosphate terminateur de chaîne au nucléotide triphosphate correspondant est compris entre 1:100 et 1:500.

5. Procédé selon l'une quelconque des revendications 1 à 4, dans lequel le micro-organisme cible est *Chlamydia trachomatis.*

6. Procédé selon la revendication 5, dans lequel la première et la seconde amorce sont choisies dans le groupe constitué par les oligonucléotides donnés par SEQ ID N° 1-17.

7. Procédé selon l'une quelconque des revendications 1 à 4, dans lequel le micro-organisme cible est le virus du sida.

8. Procédé selon la revendication 7, dans lequel la première et la seconde amorce sont choisies dans le groupe constitué par les oligonucléotides donnés par SEQ ID N° 18-20.

9. Procédé selon l'une quelconque des revendications 1 à 4, dans lequel le micro-organisme cible est le virus du papillome humain.

10. Procédé selon la revendication 9, dans lequel la première et la seconde amorce sont choisies dans le groupe constitué par les oligonucléotides donnés par SEQ ID N° 21-22.

11. Procédé selon la revendication 1, dans lequel l'échantillon d'abondance naturelle est constitué de 1 à 3 aliquotes et le nucléotide triphosphate terminateur de chaîne ajouté à chaque aliquote est différent de celui ajouté aux autres aliquotes.

12. Utilisation dans un procédé selon l'une quelconque des revendications 1 à 10 d'une composition comprenant un mélange de quatre désoxynucléotides triphosphates et d'au moins un didésoxynucléotide triphosphate correspondant à l'un des quatre désoxynucléotides triphosphates, dans lequel le didésoxynucléotide triphosphate est présent en un rapport molaire au désoxynucléotide triphosphate correspondant compris entre 1:50 et 1:500.

13. Utilisation selon la revendication 12, dans laquelle le rapport molaire est compris entre 1:100 et 1:300.

14. Utilisation selon la revendication 12 ou 13, comprenant en outre une enzyme polymérase stable à la chaleur qui incorpore des didésoxynucléotides dans un polymère acide nucléique croissant à un taux égal à au moins 0,4 fois le taux d'incorporation de désoxynucléotides.

15. Utilisation dans un procédé selon l'une quelconque des revendications 1 à 10 d'une trousse comprenant, en une combinaison emballée,
(a) une paire d'amorces qui se lient respectivement aux brins sens et antisens, et qui flanquent une région choisie dans le génome d'un micro-organisme cible, au moins l'une desdites amorces étant marquée avec un marqueur fluorescent; et
(b) un mélange de quatre désoxynucléotides triphosphates et d'au moins un didésoxynucléotide triphosphate correspondant à l'un des quatre désoxynucléotides triphosphates, le didésoxynucléotide triphosphate étant présent en un rapport molaire au désoxynucléotide triphosphate correspondant compris entre 1:50 et 1:1 000.

16. Utilisation selon la revendication 15, dans laquelle le rapport molaire est compris entre 1:100 et 1:500.

17. Utilisation selon la revendication 15 ou 16, comprenant en outre une enzyme polymérase stable à la chaleur qui incorpore des didésoxynucléotides dans un polymère acide nucléique croissant à un taux égal à au moins 0,4 fois le taux d'incorporation de désoxynucléotides.

18. Utilisation selon l'une quelconque des revendications 15 à 17, dans laquelle les amorces sont marquées chacune avec un marqueur fluorescent spectroscopiquement différent.

19. Utilisation selon l'une quelconque des revendications 15 à 18, dans laquelle le micro-organisme cible est *Chlamydia trachomatis*.

20. Utilisation selon la revendication 19, dans laquelle la première et la seconde amorce sont choisies dans le groupe constitué par les oligonucléotides donnés par SEQ ID N° 1-17.

21. Utilisation selon l'une quelconque des revendications 15 à 18, dans laquelle le micro-organisme cible est le virus du sida.

22. Utilisation selon la revendication 21, dans laquelle la première et la seconde amorce sont choisies dans le groupe constitué par les oligonucléotides donnés par SEQ ID N° 18-20.

23. Utilisation selon l'une quelconque des revendications 15 à 18, dans laquelle le micro-organisme cible est le virus du papillome humain.

24. Utilisation selon la revendication 23, dans laquelle la première et la seconde amorce sont choisis dans le groupe constitué par les oligonucléotides donnés par SEQ ID N° 21-22.
